# EUROPÄISCHE PATENTSCHRIFT

(11) **EP 0 832 603 B1**
(45) Veröffentlichungstag und Bekanntmachung des Hinweises auf die Patenterteilung: **26.03.2003**
(21) Anmeldenummer: 97114783.0
(22) Anmeldetag: 26.08.1997
(51) Int. Cl.: A61B 6/04, A61G 13/02

(54) **Patientenuntersuchungstisch**
Examination table
Table d'examen pour patient

(30) Priorität: 27.09.1996 SE 9603535
(43) Veröffentlichungstag der Anmeldung: 01.04.1998
(73) Patentinhaber: Siemens-Elema AB, 171 95 Solna (SE)
(72) Erfinder: Narfström, Jan, 191 51 Sollentuna (SE)

(56) Entgegenhaltungen:
- US-A- 2 775 496
- US-A- 5 361 436
- US-A- 5 572 569

## Beschreibung

Die Erfindung betrifft einen Untersuchungstisch für einen Patienten mit einer Lagerungsplatte und einem Bodenstativ, wobei die Lagerungsplatte in der Höhe verstellbar und kippbar ist.

Es ist von großer Bedeutung, daß die Lagerungsplatte bei Untersuchungstischen dieser Art so abgesenkt werden kann, daß sich der Patient ohne Schwierigkeiten selbst hinlegen oder von einer Bahre aus auf die Lagerungsplatte leicht herübergelegt werden kann. Danach muß die Lagerungsplatte bis zu einer Höhe, die für den Arzt akzeptabel ist und die zum Aufbau eines Röntgenstativs paßt, angehoben werden können. Ein Untersuchungstisch, der in Verbindung mit Röntgenuntersuchungen verwendet wird, kann vorzugsweise auch eine kippbare Lagerungsplatte aufweisen, insbesondere in Verbindung mit Kontrastmitteluntersuchungen, bei denen das Kontrastmittel mit Hilfe der Schwerkraft in die Richtung, in der der Patient seinen niedrigsten Punkt hat, fließt. Eine kippbare Lagerungsplatte kann auch verwendet werden, wenn der Patient sich in einem Schockzustand befindet. Die Lagerungsplatte kann dann rasch in eine Lage gekippt werden, in der der Kopf einen niedrigen Punkt erreicht. Somit kann Blut schnell zum Kopf fließen. Bei gewissen Untersuchungen ist ein Kippen der Lagerungsplatte/des Patienten von einer horizontalen in eine vertikale Lage notwendig. Ein Beispiel einer solchen Untersuchung ist es, wenn der Arzt mit dem Patienten in einer horizontalen Lage mit Hilfe einer Röntgenausrüstung Katheter in den Patienten hineinschiebt und appliziert. Danach wird die Lagerungsplatte/der Patient in eine vertikale Lage gekippt. In dieser Lage werden dann mit Hilfe einer EKG-Ausrüstung elektrophysiologische Messungen durchgeführt. Eine solche vertikale Lage ist auch in Verbindung mit gewissen Beingefässuntersuchungen interessant.

Ein Untersuchungstisch der eingangs genannten Art ist durch den Siemens-Prospekt "KOORDINAT ANGIO" bekannt. Die Lagerungsplatte ruht hier auf einem festen Bodenstativ, umfassend zwei mit Abstand voneinander angeordneten Teleskopsäulen, zwischen denen ein Röntgenfilmwechsler anbringbar ist. Bei einer Höhenverschiebung der Lagerungsplatte werden die Teleskopsäulen synchron verschoben, und beim Kippen der Platte wird die eine Teleskopsäule im Vergleich zu der anderen erhöht. Ein Kippen der Lagerungsplatte in eine vertikale Lage ist bei diesem Untersuchungstisch nicht möglich. Das Bodenstativ bei diesem Untersuchungstisch ist verhältnismäßig groß und im Aufbau kompliziert, durch der Untersuchungstisch in der Herstellung verhältnismäßig teuer wird.

In der US-PS 4 618 133 ist ein Röntgenuntersuchungstisch beschrieben, der von einer horizontalen in eine vertikale Lage gekippt werden kann. Das Bodenstativ umfaßt eine im Aufbau verhältnismäßig komplizierte, bogenförmige mit Zähnen versehene Rahmenkonstruktion und kann daher mit Hilfe von motorgetriebenen Zahnrädern und Kettentransmissionen den Tisch in beschriebener Weise kippen. Die Lagerungsplatte ist nicht in der Höhe verstellbar. Außerdem erstreckt sich das Bodenstativ über die gesamte Länge der Lagerungsplatte, was mitsichbringt, daß der Untersuchungstisch platzraubend ist.

Der Erfindung liegt die Aufgabe zugrunde, einen Untersuchungstisch der eingangs genannten Art zu schaffen mit einem Bodenstativ, das im Aufbau extrem einfach und platzsparend ist und das außerdem ein Kippen der Lagerungsplatte in eine vertikale Lage erlaubt.

Diese Aufgabe ist erfindungsgemäss dadurch gelöst, daß. das Bodenstativ einen bogenförmigen Balken aufweist, bei dem das eine Ende des Balkens am Boden um eine horizontale Welle drehbar befestigt ist und wobei die Lagerungsplatte mit einer Halterung versehen ist, die an dem Balken derart verschiebbar angebracht ist, daß die Halterung mit der Lagerungsplatte in der Längsrichtung des Balkens verschoben werden kann. Durch Drehen des Balkens um die horizontale Achse gleichzeitig damit, daß die Halterung mit der Lagerungsplatte in der Längsrichtung des Balkens verschoben werden kann, kann die Lagerungsplatte in die Höhe verstellt und gekippt werden, auch bis die Platte eine vertikale Lage einnimmt.

In der DE 4 229 318 C1 ist ein Röntgenuntersuchungstisch beschrieben mit einer Lagerungsplatte, die dazu vorgesehen ist, um einen im Raum fiktiven festen Punkt geschwenkt zu werden. Der Untersuchungstisch umfaßt ein Bodenstativ mit einem bogenförmigen Balken, bei dem der Bogen nach oben gerichtet ist, und der in einer am Boden angebrachten Halterung verschiebbar und mit der Lagerungsplatte über eine horizontale Welle drehbar verbunden ist. Der Balken ist derart ausgebildet, daß er unterhalb der und parallel zur Lagerungsplatte verläuft, so daß die Lagerungsplatte lediglich in die eine Richtung gekippt werden kann. Bei einem Herabsenken der Lagerungsplatte wird der Balken derart verschoben, daß er an der einen Seite der Halterung nach oben herausragt und dadurch störend sein kann. Außerdem wird die Lagerungsplatte bei einer Höhenverschiebung in ihrer Längsrichtung verschoben.

In einer vorteilhaften Weiterentwicklung des Untersuchungstisches nach der Erfindung wird vorgeschlagen, daß der Balken die Form einer halben Ellipse, die entlang deren großen Achse geschnitten ist, oder eines Teils dieser halben Ellipse aufweist. Durch die Form des Balkens kann die Lagerungsplatte in eine Position herabgesenkt werden, die für eine Lagerung eines Patienten günstig ist und in eine Höhenlage gefahren werden, die für den Arzt eine optimale Arbeitsposition ist.

Der Balken kann nach der Erfindung auch ein Kreisbogensegment mit einem Sektorwinkel bis zu 180° aufweisen.

Die Lagerungsplatte kann nach der Erfindung vorzugsweise in ihrer Längsrichtung verschoben werden.

Die Erfindung ist nachfolgend anhand zwei in den Zeichnungen dargestellten Ausführungsbeispielen näher erläutert. Es zeigen:

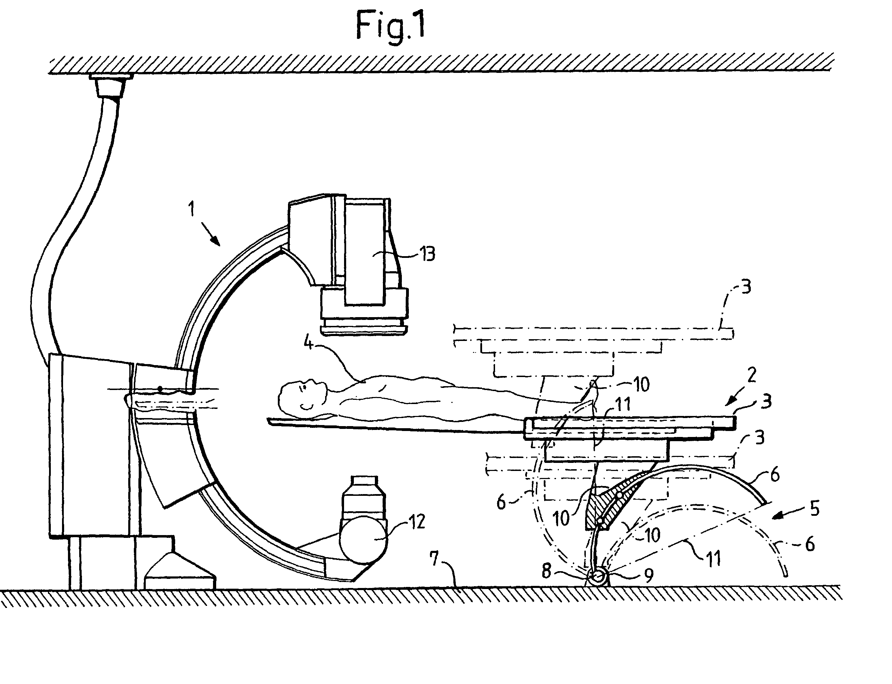

FIG. 1 bis 3 Seitenansichten von Untersuchungstischen nach der Erfindung in zwei Ausführungen und in verschiedenen Lagen.

In der FIG. 1 ist ein Stativsystem mit einem Röntgenstativ 1 und einem Untersuchungstisch 2 nach der Erfindung gezeigt. Der Untersuchungstisch 2 umfaßt eine Lagerungsplatte 3, auf der ein Patient 4 gelagert ist, und ein Bodenstativ 5. Das Bodenstativ 5 weist einen bogenförmigen Balken 6 auf, wobei das eine Ende des Balkens 6 am Boden 7 um eine horizontale Welle 8, die mit einem Motor 9 verbunden ist, drehbar befestigt ist. Die Lagerungsplatte 3 ist mit einer Halterung 10 versehen, in der der Balken 6 angebracht ist. Der Balken 6 in diesem Ausführungsbeispiel hat die Form einer halben Ellipse, die entlang deren großen Achse 11 geschnitten ist. Die große Achse 11 ist in dieser FIG mit einer geraden strichpunktierten Linie gezeigt. Die Lagerungsplatte 3 ist vorzugsweise auch in ihrer Längsrichtung verschiebbar.

Die Halterung 10 mit der Lagerungsplatte 3 ist in die Längsrichtung des Balkens 6 mit Hilfe eines Motors, Rollen und Zahnriemen, die in der Halterung 10 angebracht sind, verschiebbar. Solche Mittel zum Verschieben sind in Verbindung mit Röntgenstativen, die einen C-förmigen Bogen aufweisen und der in einer Halterung verschiebbar gelagert ist, aufweisen, bekannt. Ein Beispiel eines solchen Röntgenstativs ist aus dem Siemens-Prospekt "The fast and flexible new T.O.P.-Line Cardiac cath lab COROSKOP T.O.PIBICOR T.O.P." bekannt.

In der FIG. 1 ist gezeigt, daß der Balken 6 mit Hilfe des Motors 9 um die Welle 8 gedreht und die Halterung 10 auf dem Balken 6 in eine Lage verschoben worden ist, in der die Lagerungsplatte 3 mit dem Patienten eine Höhenlage erreicht hat, die für eine Röntgenuntersuchung geeignet ist. In der FIG. ist auch dargestellt, daß die Lagerungsplatte 3 mit bekannten und daher nicht näher beschriebenen Mitteln in ihrer Längsrichtung bis zu einer Lage verschoben worden ist, in der die Brust des Patienten zwischen der Röntgenröhre 12 und dem Bildverstärker 13 des Röntgenstativs zu liegen kommt. Die in der FIG. strichpunktierte Kontur des Balkens 6, wo dieser in eine Lage gedreht worden ist, in der dessen freies Ende annähernd gegen den Boden 7 anliegt und in der die ebenfalls strichpunktierten Halterung 10 am Balken in Richtung der Welle 8 verschoben worden ist, zeigt eine niedrigste Lage der Lagerungsplatte 3. In der FIG. ist auch eine strichpunktierte Kontur des Balkens 6 in einer Lage gezeigt, in der die große Achse 11 der Ellipse annähernd senkrecht zum Boden 7 ist und die Halterung 10 gleichzeitig am Balken 6 nach oben gefahren worden ist. Hier hat die Lagerungsplatte ihre höchste Lage erreicht. Eine Höhenverschiebung der Lagerungsplatte 3 von einer beschriebenen niedrigsten Lage in eine beschriebene höchste Lage mit einer beibehaltenden vertikalen Position kann erreicht werden, indem der Motor 9 den Balken 6 um die Welle 8 dreht gleichzeitig damit, daß die Halterung 10 mit Hilfe des hier nicht gezeigten Motors in der Längsrichtung des Balkens 6 verschoben wird. Vorrichtungen zur Steuerung von Motoren wie es in diesem Ausführungsbeispiel beschrieben worden ist, sind durch die Robottechnik bekannt und werden daher nicht in der Anmeldung näher angegeben.

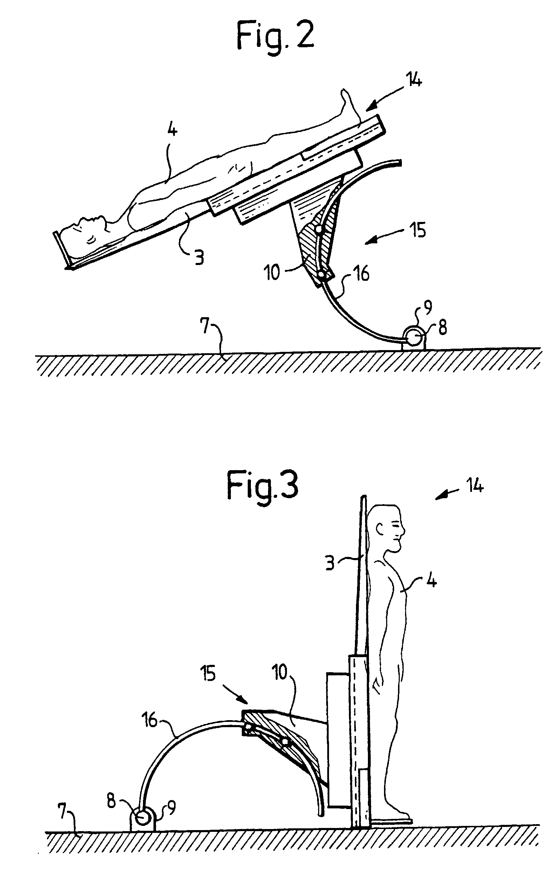

In der FIG 2 ist eine weitere Ausführungsform eines Untersuchungstisches 14 nach der Erfindung gezeigt. Der Untersuchungstisch 14 unterscheidet sich von dem in der FIG 1 gezeigten Untersuchungstisch 2 lediglich dadurch, daß das Bodenstativ 15 einen Balken 16 umfaßt, der ein Kreisbogensegment mit einem Sektorwinkel von 180 aufweist. Der Sektorwinkel kann auch kleiner als 180 sein. Im übrigen ist der Untersuchungstisch 14 auf die gleiche Weise wie der in der FIG 1 ausführlich beschriebene Untersuchungstisch 2, aufgebaut.

In der FIG 2 ist gezeigt, daß der Balken 16 mittels des Motors 9 um die Welle 8 gedreht und die Halterung mit beschriebenen Mitteln in Längsrichtung des Balkens 16 in eine Lage gefahren werden kann, in der die Lagerungsplatte 3 mit dem Patienten 4 nach vorne gekippt wird, so daß der Kopf des Patienten 4 eine im Vergleich zum Körper niedrigste Lage einnimmt.

In der FIG. 3 ist gezeigt, daß der Balken 16 mittels des Motors 9 um die Welle 8 in eine Lage gedreht worden ist, in der das freie Ende des Balkens 16 fest am Boden 7 anliegt und die Halterung 10 in einer Lage, in der die Lagerungsplatte 3 und damit auch der Patient eine zum Boden annähernd senkrechte Lage eingenommen hat, verschoben worden ist.

Auch der in Verbindung mit der FIG 1 beschriebene Untersuchungstisch 2 nach der Erfindung kann mit Hilfe des ellipsförmigen Balkens 6 und der Halterung 10 die Lagerungsplatte 3 in der Weise, wie es in den FIG 2 und 3 gezeigt worden ist, positioniert werden. Der ellipsförmige Balken 6 kann im übrigen ein Teil dieser halben Ellipse sein und braucht demzufolge keine volle halbe Ellipse zu sein. Auch der Untersuchungstisch 14 kann auf dieselbe Weise wie es in Verbindung mit dem Untersuchungstisch 2 beschrieben und in der FIG 1 gezeigt worden ist, in der Höhe verstellt werden. Darüber hinaus kann die Lagerungsplatte 3 im Rahmen der Erfindung in beschriebener Weise nach Wunsch des Arztes in weitere in Verbindung mit unterschiedlichen Untersuchungen, in den FIG nicht gezeigte Positionen gefahren werden. Zusammenfassend ist durch die Erfindung ein im Aufbau sehr einfacher und dadurch in der Herstellung verhältnismäßig billiger in der Höhe verstellbarer und kippbarer Untersuchungstisch gegeben, der insbesondere durch die Form und Ausführung des Bodenstativs extrem platzsparend ist.

### Bezugszeichenliste

- 1: Röntgenstativ
- 2, 14: Untersuchungstisch
- 3: Lagerungsplatte
- 4: Patient
- 5, 15: Bodenstativ
- 6, 16: bogenförmiger Balken
- 7: Boden
- 8: Welle
- 9: Motor
- 10: Halterung
- 11: große Achse
- 12: Röntgenröhre
- 13: Bildverstärker

## Patentansprüche

1. Untersuchungstisch für einen Patienten mit einer Lagerungsplatte und einem Bodenstativ, wobei die Lagerungsplatte in der Höhe verstellbar und kippbar ist, **dadurch gekennzeichnet, daß** das Bodenstativ einen bogenförmigen Balken (6, 16) aufweist, bei dem das eine Ende des Balkens (6, 16) am Boden (7) um eine horizontale Welle (8) drehbar befestigt ist und wobei die Lagerungsplatte (3) mit einer Halterung (10) versehen ist, die an dem Balken (6, 16) derart verschiebbar angebracht ist, daß die Halterung (10) mit der Lagerungsplatte (3) in der Längsrichtung des Balkens (6, 16) verschoben werden kann.

2. Untersuchungstisch nach Anspruch 1, **dadurch gekennzeichnet, daß** der Balken (6) die Form einer halben Ellipse, die entlang deren großen Achse (11) geschnitten ist, oder eines Teils dieser halben Ellipse (6) aufweist.

3. Untersuchungstisch nach Anspruch 1, **dadurch gekennzeichnet**, das der Balken (16) ein Kreisbogensegment mit einem Sektorwinkel bis zu 180° aufweist.

4. Untersuchungstisch nach einem der Ansprüche 1 bis 3, **dadurch gekennzeichnet, daß** die Lagerungsplatte (3) in ihrer Längsrichtung verschiebbar ist.

## Claims

1. Examination table for a patient, with a supporting panel and a floor stand, the supporting panel being vertically adjustable and tiltable, **characterized in that** the floor stand has an arc-shaped beam (6, 16), one end of the beam (6, 16) being secured to the floor (7) in a rotatable manner about a horizontal shaft (8), and the supporting panel (3) being provided with a mounting (10) which is arranged on the beam (6, 16) in a displaceable manner so that the mounting (10) with the supporting panel (3) can be displaced in the longitudinal direction of the beam (6, 16).

2. Examination table according to Claim 1, **characterized in that** the beam (6) has the shape of a half ellipse, which is cut along its main axis (11), or of a part of this half ellipse (6).

3. Examination table according to Claim 1, **characterized in that** the beam (16) has a circle arc segment with a sector angle of up to 180°.

4. Examination table according to one of Claims 1 to 3, **characterized in that** the supporting panel (3) is displaceable in its longitudinal direction.

## Revendications

1. Table d'examen pour un patient comprenant un plateau servant de couchette et un pied, le plateau servant de couchette étant réglable en hauteur et pouvant être basculé, **caractérisée en ce que** le pied comprend une poutrelle (6,16) en forme d'arc, l'une des extrémités de la poutrelle (6,16) étant fixée au sol (7) avec possibilité de toumer par rapport à un arbre (8) horizontal et le plateau (3) formant couchette étant muni d'une fixation (10) qui est montée coulissante sur la poutrelle (6,16) de façon à ce que la fixation (10) puisse être déplacée avec le plateau (3) formant couchette dans la direction longitudinale de la poutrelle (6,16).

2. Table d'examen suivant la revendication 1, **caractérisée en ce que** la poutrelle (6) a la forme d'une demi-ellipse qui est coupée le long de son grand axe (11) ou d'une partie de cette demi-ellipse (6).

3. Table d'examen suivant la revendication 1, **caractérisée en ce que** la poutrelle (16) comporte un segment d'arc de cercle ayant un angle de secteur allant jusqu'à 180°.

4. Table suivant l'une des revendications 1 à 3, **caractérisée en ce que** le plateau (3) formant couchette peu coulisser dans sa direction longitudinale.
